(19) Europäisches Patentamt
European Patent Office
Office européen des brevets

(11) **EP 1 304 542 B1**

(12) **EUROPÄISCHE PATENTSCHRIFT**

(45) Veröffentlichungstag und Bekanntmachung des Hinweises auf die Patenterteilung:
**02.04.2014 Patentblatt 2014/14**

(51) Int Cl.:
*G01B 7/00* *(2006.01)*    *A61B 5/06* *(2006.01)*
*A61B 5/0275* *(2006.01)*

(21) Anmeldenummer: **02102459.1**

(22) Anmeldetag: **17.10.2002**

(54) **Verfahren zur Ermittlung der räumlichen Verteilung magnetischer Partikel**

Method for determining the spacial distribution of magnetic particles

Méthode pour déterminer la distribution spatiale de particules magnétiques

(84) Benannte Vertragsstaaten:
**DE FR GB**

(30) Priorität: **19.10.2001 DE 10151778**

(43) Veröffentlichungstag der Anmeldung:
**23.04.2003 Patentblatt 2003/17**

(73) Patentinhaber:
• **Philips Intellectual Property & Standards GmbH 20099 Hamburg (DE)**
Benannte Vertragsstaaten:
**DE**
• **Koninklijke Philips N.V.
5656 AE Eindhoven (NL)**
Benannte Vertragsstaaten:
**FR GB**

(72) Erfinder: **Gleich, Bernhard
D-52088 Aachen (DE)**

(74) Vertreter: **Verweij, Petronella Danielle et al
Philips Intellectual Property & Standards
P.O. Box 220
5600 AE Eindhoven (NL)**

(56) Entgegenhaltungen:
**WO-A-01/40790    GB-A- 1 070 729
US-A- 4 136 683    US-A- 4 238 734
US-A- 4 849 340    US-A- 5 384 109
US-A- 5 393 525    US-A- 5 794 622
US-B1- 6 168 780**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung betrifft ein Verfahren zur Ermittlung der räumlichen Verteilung magnetischer Partikel in einem Untersuchungsbereich. Außerdem betrifft die Erfindung die Verwendung geeigneter magnetischer Partikel für ein solches Verfahren und eine Anordnung zur Durchführung des Verfahrens.

Magnetische Stoffe lassen sich verhältnismäßig einfach detektieren und lassen sich deshalb für - insbesondere medizinische - Untersuchungen einsetzen.

So ist aus der DE-PS 19 532 676 ein Verfahren zur Bestimmung der Position eines magnetischen Markers im Magen-Darm-Trakt bekannt. Bei diesem Verfahren wird ein einzelner magnetischer Marker mit einem Durchmesser von ca. 8 mm in den Magen-Darm-Trakt eingebracht. Um die Passage dieses Markers im Magen-Darm-Trakt zu verfolgen, wird er in einer bestimmten zeitlichen Folge einem impulsweise wirksamen äußeren Magnetfeld mit wechselnder Polarität ausgesetzt, wodurch der Marker während seiner Passage wiederholt aufmagnetisiert wird, wodurch sich sein magnetisches Moment jeweils neu parallel zu dem äußeren Magnetfeld einstellt.

Das von dem Marker stammende Sekundärmagnetfeld wird mittels anisotroper Magnetfeldsensoren parallel und senkrecht zur Achse der das äußere Magnetfeld erzeugenden Spule getrennt vermessen, wobei die Spule mit den daran befestigten Magnetfeldsensoren so lange verschoben wird, bis die Magnetfeldsensoren ein Null-Signal liefern. Die auf diese Weise sich ergebende Position der Spule ist mit der Position des Markers im Magen-Darm-Trakt korreliert. In Verbindung mit dem jeweiligen Messzeitpunkt lässt sich auf diese Weise die Bewegung des Markers ermitteln. Diese Verfahren hat eine geringe räumliche und zeitliche Auflösung.

Aus der WO 01/40 790 A1 ist ein Verfahren und eine Vorrichtung zur quantitativen Messung von Anhäufungen magnetischer Partikel bekannt. Die magnetischen Partikel werden dabei von einem äußeren Magnetfeld derart angeregt, dass sie in der Anregungsfrequenz schwingen und ähnlich wie ein Dipol ihre eigenen Felder erzeugen. Durch induktive Messung der Amplituden der resultierenden Oszillationen, lassen sich Rückschlüsse auf die Anwesenheit und die Konzentration der magnetischen Partikel ziehen.

Aus der US 4,136,683 A ist ein Verfahren zur intrazellulären Temperaturmessung bekannt. Hierbei werden magnetische Partikel in das Zellinnere der zu bestimmenden Zellen eingeführt. Durch Messung der magnetischen Eigenschaften wird dann die Temperatur der magnetischen Partikel und damit auch die Temperatur des Zellinneren bestimmt.

Aus der US 5,384,109 A ist ferner ein Verfahren zur diagnostischen Magnetometrie mit Hilfe eines SQUID-Magnetometers bekannt, bei welchem zur Bilderzeugung ein paramagnetisches bzw. superparamagnetisches Kontrastmittel verwendet wird.

Weiterhin sind MR-Verfahren (MR=Magnetresonanz) bekannt, bei denen ferro- oder ferrimagnetische Partikel in die Blutbahn eines Patienten injiziert werden, um den Kontrast der

Blutgefäße anzuheben. Die Partikel sind dabei so klein (5 bis 10 nm), dass sich darin keine Weiß'schen Bereiche ausbilden können. Ein Nachteil von MR-Verfahren sind die hohen Kosten für ein MR-Gerät zur Durchführung des MR-Verfahrens. Unter anderem benötigt ein solches MR-Gerät einen Magneten, der im Untersuchungsbereich während der gesamten MR-Untersuchung ein homogenes stationäres Magnetfeld erzeugt. Um ein ausreichendes Signal-/Rausverhältnis erzielen zu können, muss dieses Magnetfeld eine Stärke von 0,5 Tesla oder mehr haben. Dafür sind supraleitende Magnete erforderlich.

Aufgabe der Vorliegenden Erfindung ist es, ein Verfahren zur Ermittlung der räumlichen Verteilung magnetischer Partikel in einem Untersuchungsbereich anzugeben, das eine gute zeitliche und räumliche Auflösung aufweist und einen vergleichsweise niedrigen apparativen Aufwand zu seiner Durchführung benötigt.

Diese Aufgabe wird gelöst durch ein Verfahren zur Ermittlung der räumlichen Verteilung magnetischer Partikel in einem Untersuchungsbereich mit den Schritten

a) Erzeugung eines Magnetfeldes mit einem solchen räumlichen Verlauf der magnetischen Feldstärke, dass sich in dem Untersuchungsbereich ein erster Teilbereich (301) mit niedriger magnetischer Feldstärke ergibt, in welchem die Magnetisierung der magnetischen Partikel nicht gesättigt ist, und ein zweiter Teilbereich (302) mit höherer magnetischer Feldstärke ergibt, in welchem die Magnetisierung der magnetischen Partikel im Zustand der Sättigung ist,

b) Veränderung der räumlichen Lage der beiden Teilbereiche in dem Untersuchungsbereich, so dass die Magnetisierung der Partikel sich örtlich ändert,

c) Erfassung von Signalen, die von der durch die Veränderung der räumlichen Lage beeinflussten Magnetisierung im Untersuchungsbereich abhängen,

d) Auswertung der Signale zur Gewinnung von Information über die räumliche Verteilung der magnetischen Partikel im Untersuchungsbereich.

Bei der Erfindung wird im Untersuchungsbereich ein räumlich inhomogenes Magnetfeld erzeugt. In dem ersten Teilbereich ist das Magnetfeld so schwach, dass die Magnetisierung der Partikel mehr oder weniger stark vom äußeren Magnetfeld abweicht, also nicht gesättigt ist. Dieser erste Teilbereich ist vorzugsweise ein räumlich zusammenhängender Bereich; er kann ein punktförmiger Bereich sein, aber auch eine Linie oder eine Fläche. In dem zweiten Teilbereich (d. h. in dem außerhalb des ersten Teils verbleibenden Rest des Untersuchungs-bereichs) ist das Magnetfeld genügend stark, um die Partikel in einem Zustand der Sättigung zu halten. Die Magnetisierung ist gesättigt, wenn die Magnetisierung nahezu aller Partikel in ungefähr der Richtung des äußeren Magnetfeldes ausgerichtet ist, so dass mit einer weiteren Erhöhung des Magnetfeldes die Magnetisierung dort wesentlich weniger zunimmt als im ersten Teilbereich bei einer entsprechenden Erhöhung des Magnetfeldes.

Durch Veränderung der Lage der beiden Teilbereiche innerhalb des Untersuchungs-bereichs ändert sich die (Gesamt-)Magnetisierung im Untersuchungsbereich. Misst man daher die Magnetisierung im Untersuchungsbereich oder davon beeinflusste physikalische Parameter, dann kann man daraus Informationen über die räumliche Verteilung der magnetischen Partikel im Untersuchungsbereich ableiten.

In der Praxis weisen die Partikel nicht identische magnetische Eigenschaften auf. Beispielsweise kann sich ein Teil der Partikel bei einer magnetischen Feldstärke in Sättigung befinden, bei der sich ein anderer Teil noch im Zustand der Nicht-Sättigung befindet. Jedoch ergibt sich hierdurch eine (zusätzliche) Nichtlinearität in der Magnetisierungskennlinie, die bei einer Veränderung der Lage der beiden Teilbereiche zu einer Änderung der Magnetisierung im Untersuchungsbereich führt.

Eine Möglichkeit zur Veränderung der räumlichen Lage der beiden Teilbereiche besteht darin, dass eine zur Erzeugung des Magnetfeldes vorgesehene Spulen- und/oder Permanentmagnet-Anordnung (oder Teile davon) einerseits oder das Untersuchungsobjekt andrerseits relativ zueinander verschoben werden. Dies ist eine bevorzugte Methode wenn mit sehr starken Gradienten sehr kleine Objekte untersucht werden (Mikroskopie).

Demgegenüber beschreibt Anspruch 2 eine bevorzugte Ausgestaltung, die keine mechanischen Bewegungen erfordert. Die räumliche Lage der beiden Teilbereiche lässt sich dabei relativ schnell verändern, was zusätzliche Vorteile bei der Erfassung von Signalen bietet, die von der Magnetisierung im Untersuchungsbereich abhängen.

Bei der Ausgestaltung nach Anspruch 3 werden Signale erfasst, die der zeitlichen Änderung der Magnetisierung im Untersuchungsbereich proportional sind. Damit diese Signale möglichst groß sind, ist es wichtig, dass die räumliche Lage der beiden Teilbereiche im Untersuchungsbereich möglichst schnell verändert wird. Zur Erfassung dieser Signale kann eine Spule benutzt werden, mit der im Untersuchungsbereich ein Magnetfeld erzeugt wird Vorzugsweise wird aber eine gesonderte Spule benutzt.

Die Veränderung der räumlichen Lage der Teilbereiche kann mittels eines zeitlich veränderlichen Magnetfeldes vonstatten gehen. Dabei wird in einer Spule ein ebenfalls periodisches Signal induziert. Der Empfang dieses Signals gestaltet sich aber insofern schwierig, als die im Untersuchungsbereich erzeugten Signale und das zeitlich veränderliche gleichzeitig wirksam sind; es kann daher nicht ohne weiteres zwischen den durch das Magnetfeldes induzierten Signalen und den durch Änderung der Magnetisierung im Untersuchungsbereich induzierten Signalen unterschieden werden.

Bei der Ausgestaltung nach Anspruch 4 wird dieses Problem vermieden. Dabei wird die Tatsache ausgenutzt, dass die Frequenzkomponenten des zweiten Frequenzbandes nur durch eine Änderung der Magnetisierung im Untersuchungsbereich infolge der Nichtlinearität der Magnetisierungskennlinie entstehen können. Wenn das zeitlich veränderlichen Magnetfeld dabei einen sinusförmigen periodischen Verlauf hat, besteht das erste Frequenzband nur aus einer einzigen Frequenzkomponente - der sinusförmigen Grundschwingung Hingegen enthält das zweite Frequenzband neben dieser Grundschwingung auch höhere Harmonische (sog. Oberwellen) der sinusförmigen Grundschwingung, die zur Auswertung herangezogen werden können.

Die magnetischen Partikel, die für das erfindungsgemäße Verfahren geeignet sind, müssen Abmessungen haben, die klein gegenüber der Größe der Voxel sind, deren Magnetisierung durch das erfindungsgemäße Verfahren ermittelt werden soll. Weiterhin muss die Magnetisierung der Partikel bei möglichst geringen Feldstärken des Magnetfeldes in die Sättigung gelangen. Je geringer die dafür erforderliche Feldstärke ist, desto höher ist das räumliche Auflösungsvermögen bzw. desto schwächer kann das im Untersuchungsbereich zu erzeugende (externe) Magnetfeld sein. Weiterhin sollen die magnetischen Partikel ein möglichst hohes Dipol-Moment bzw. eine hohe Sättigungsinduktion haben, damit die Änderung der Magnetisierung möglichst große Ausgangssignale zur Folge hat. Beim Einsatz des Verfahrens für medizinische Untersuchungen ist darüber hinaus wichtig, dass die Partikel nicht toxisch sind

Bei der Ausgestaltung nach Anspruch 5 sind die Partikel so klein, dass sich in ihnen nur eine einzige magnetische Domäne (die Monodomäne) ausbilden kann bzw. keine Weiß'schen Bereiche entstehen können. Die Abmessungen der Partikel müssen dabei im Nanometerbereich liegen. Bei den eingangs erwähnten Kontrastmitteln für MR-Untersuchungen haben diese Partikel eine Größe von 5 bis 10 nm. Diese Partikelgröße ist für die Erfindung noch nicht optimal. Bei größeren Abmessungen der Partikel können kleinere Feldstärken ausreichen, um eine Sättigung der Magnetisierung der Partikel zu gewährleisten. Jedoch dürfen die Abmessungen nicht so groß werden, dass sich in den Partikeln mehrere magnetische Domänen bzw. Weiß'sche Bereiche ausbilden können. Geeignete Partikelgrößen liegen daher in einem Bereich von 20 nmbis ca. 800 nm, wobei die obere Grenze auch von dem Material abhängt. Ein für Monodomänen-Partikel geeignetes Material ist beispielsweise Magnetit ($Fe_3O_4$). Solche Partikel können z. B. für Lungenuntersuchungen inhaliert werden.

Bei der Ausgestaltung nach Anspruch 6 werden demgegenüber größere Partikel verwendet, in denen sich eine Anzahl magnetischer Domänen ausbilden kann. Im Hinblick auf das räumliche Auflösung sollten diese Partikel aus einem magnetischen Material bestehen, das bei niedriger magnetischer Feldstärke in Sättigung ist (was eine niedrige Sättigungsinduktion voraussetzt). Diese Voraussetzung kann bei der Weiterbildung nach Anspruch 7 entfallen. Weil die Partikel dabei nur eine dünne Schicht aus magnetischem Material aufweisen, ist auch dann eine magnetische Sättigung bei niedriger Feldstärke gewährleistet, wenn die Schicht nicht aus einem Material mit niedriger Sättigungsinduktion besteht.

Die Ausgestaltung nach Anspruch 8 erlaubt es, die Partikel bei medizinischen Untersuchungen auf einfache Weise zu applizieren. Benutzt man eine Dispersion mit den Monodomänen-Partikeln gemäß Anspruch 5, dann kann diese Dispersion in die Blutbahn injiziert werden, um beispielsweise den Gefäßbaum oder das Herz darzustellen. Diese Applikation ist nicht toxisch, wie die Verwendung der erwähnten MR-Kontrastmittel gezeigt hat. Eine Dispersion mit den Anspruch 6 oder 7 definierten Partikeln kann - nach der oralen Einnahme durch einen zu untersuchenden Patienten - zur Untersuchung des Magen-Darm-Traktes verwendet werden.

Im allgemeinen ist es von Vorteil, wenn die Partikel eine niedrige effektive Anisotropie aufweisen (unter "effektiver Anisotropie" wird hierbei und im folgenden die aus der Form-Anisotropie und aus der Kristall- Anisotropie resultierende magnetische Anisotropie verstanden), weil eine Änderung ihrer Magnetisierungsrichtung keine Drehung dieser Partikel erfordert. Deshalb können auch schnell veränderliche Magnetfelder verwendet werden, wodurch sich höhere Signalamplituden und ein günstigeres Signal/Rausch-Verhältnis ergibt. Demgegenüber kann es ebenso ausgenutzt werden, dass bei Partikeln mit genügend großer effektiver Anisotropie (beispielsweise länglichen Partikeln) eine Änderung der Magnetisierungsrichtung eine mechanische Drehung der Partikel voraussetzt. Die Geschwindigkeit, mit der diese Richtungsänderung in einem flüssigen Medium erfolgen kann, ist ein Maß für die Viskosität in diesem Medium.

Eine Anordnung zur Durchführung des erfindungsgemäßen Verfahrens ist in Anspruch 9 angegeben. Bei der bevorzugten Ausgestaltung dieser Anordnung nach Anspruch 10 ist eine Gradienten-Spulenanordnung zur Erzeugung des Magnetfeldes im Untersuchungsbereich vorgesehen. Dieses Magnetfeld ist - wenn die Gradienten-Spuleanordnung z. B. zwei beiderseits des Untersuchungsbereichs angeordnete gleichartige, aber von gegensinnigen Strömen durchflossene, Wicklungen umfasst (Maxwellspule) - an einem Punkt auf der Wicklungsachse Null und nimmt beiderseits dieses Punktes mit entgegengesetzter Polarität nahezu linear zu. Nur bei den Partikeln, die sich im Bereich um diesen Feld-Nullpunkt befinden, ist die Magnetisierung nicht gesättigt. Bei den Partikeln außerhalb dieses Bereiches ist die Magnetisierung im Zustand der Sättigung.

Bei der Weiterbildung nach Anspruch 11 wird der von der Gradienten-Spulenanordnung erzeugte Bereich um den Feld-Nullpunkt herum, d.h. der erste Teilbereich, innerhalb des Untersuchungsbereichs durch das zeitlich veränderliche Magnetfeld verschoben. Bei geeignetem zeitlichen Verlauf und Orientierung dieses Magnetfeldes kann auf diese Weise der Feld-Nullpunkt den gesamten Untersuchungsbereich durchlaufen.

Die mit der Verschiebung des Feld-Nullpunktes einhergehende Magnetisierungsänderung kann entsprechend der Weiterbildung nach Anspruch 12 detektiert werden. Die zum Empfang der im Untersuchungsbereich erzeugten Signale benutzte Spule kann dabei eine Spule sein, die bereits zur Erzeugung des Magnetfelds im Untersuchungsbereich dient. Es hat jedoch auch Vorteile, zum Empfang eine gesonderte Spule zu verwenden, weil diese von der Spulenanordnung entkoppelt werden kann, die ein zeitlich veränderliches Magnetfeld erzeugt. Außerdem kann mit einer Spule - erst recht aber mit mehreren Spulen - ein verbessertes Signal/Rausch-Verhältnis erzielt werden.

Die Amplitude der in der Spulenanordnung induzierten Signale ist umso größer, je schneller sich die Position des Feld-Nullpunkt im Untersuchungsbereich ändert, d. h. je schneller sich das dem magnetischen Gradientenfeld überlagerte zeitlich veränderliche Magnetfeld ändert. Es ist aber technisch schwierig, einerseits ein zeitlich veränderliches Magnetfeld zu erzeugen, dessen Amplitude ausreicht, um den Feld-Nullpunkt an jeden Punkt des Untersuchungsbereichs zu verschieben und dessen Änderungsgeschwindigkeit genügend groß ist, um Signale mit einer ausreichenden Amplitude zu erzeugen. Dieses Problem wird durch die Ausgestaltung nach Anspruch 13 entschärft, bei der zwei unterschiedlich schnell und mit unterschiedlicher Amplitude veränderliche Magnetfelder - vorzugsweise von zwei Spulenanordnungen - erzeugt werden. Als weiterer Vorteil ergibt sich, dass die Feldänderungen so schnell sein können (z.B> 20 kHz), dass sie oberhalb der menschlichen Hörgrenze liegen.

Die weitere Ausgestaltung nach Anspruch 14 erlaubt die Verschiebung des feldfreien Punktes in einem zweidimensionalen Bereich. Durch ein weiteres Magnetfeld, das eine Komponente besitzt, die senkrecht zu den beiden Magnetfeldern verläuft, ergibt sich eine Erweiterung auf einen dreidimensionalen Bereich.

Bei der Ausgestaltung nach Anspruch 15 wird die Tatsache ausgenutzt, dass die Magnetisierungs-Kennlinie in dem Bereich, in dem die Magnetisierung von dem nicht gesättigten in den gesättigten Zustand übergeht, nichtlinear ist. Diese Nichtlinearität bewirkt, dass ein z.B. ein zeitlich sinusförmig verlaufendes Magnetfeld mit der Frequenz f im Bereich der Nichtlinearität eine zeitlich veränderliche Induktion mit der Frequenz f (Grundwelle) und ganzzahligen Vielfachen der Frequenz f (Oberwellen bzw. höhere Harmonische) hervorruft. Die Auswertung der Oberwellen hat den Vorteil, dass die Grundwelle des gleichzeitig zur Verschiebung des feldfreien Punktes wirksamen Magnetfeldes keinen Einfluss auf die Auswertung hat.

Die Erfindung wird nachstehend anhand von Zeichnungen näher erläutert. Es zeigen:

Fig. 1 ein Gerät zur Durchführung des erfmdungsgemäßen Verfahrens,
Fig. 2 den durch eine der darin enthaltenen Spulen erzeugten Feldlinienverlauf,
Fig. 3 eines der im Untersuchungsbereich vorhandenen magnetischen Partikel,
Fig. 4 die Magnetisierungskennlinie derartiger Partikel,
Fig. 5 ein Prinzipschaltbild der Anordnung nach Fig. 1,
Fig. 6 den Verlauf verschiedener Signale bei dem Gerät nach den Fig. 1 und 5, und
Fig. 7 die Verschiebung des feldfreien Punktes in einem zweidimensionalen Bereich.

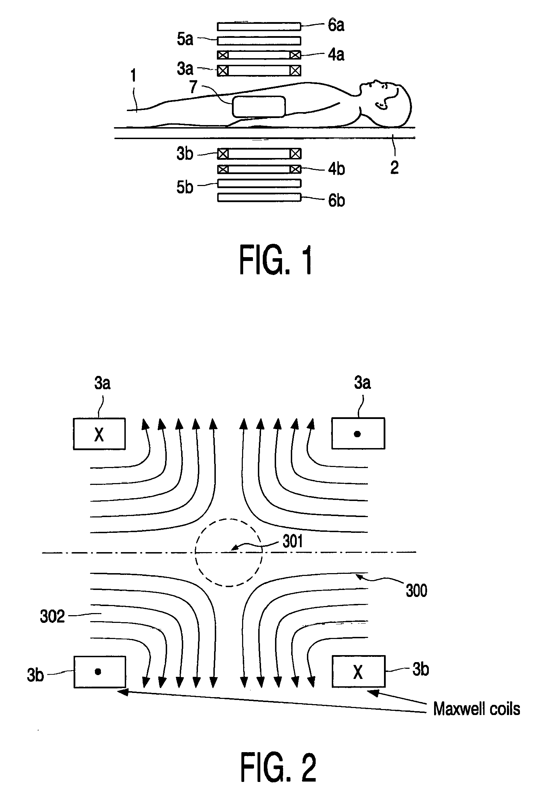

In Fig. 1 ist 1 ein Untersuchungsobjekt bezeichnet, in diesem Fall ein Patient, der sich auf einem Patientenlagerungstisch befindet, von dem lediglich die Tischplatte 2 teilweise angedeutet ist. Vor einer Untersuchung beispielsweise des Magen-Darm-Traktes wird dem Patienten 1 eine Flüssigkeit oder ein Brei mit magnetischen Partikeln verabreicht.

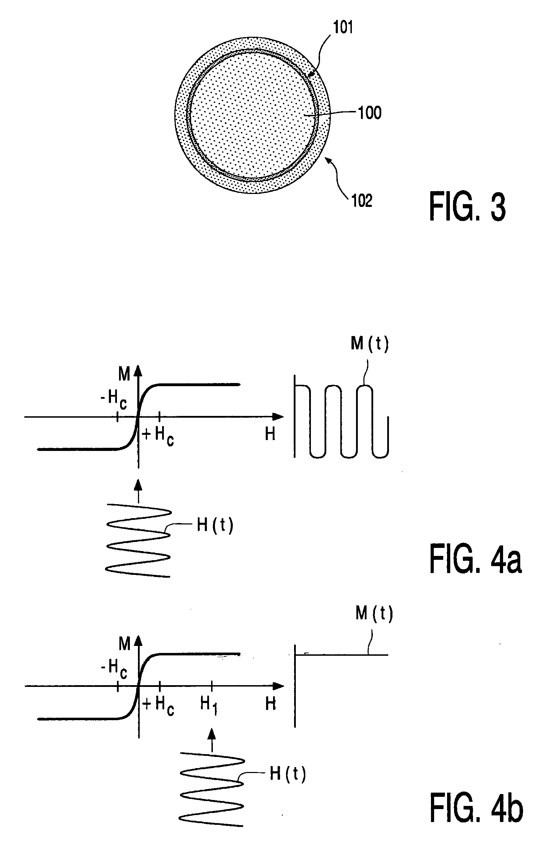

Ein solches Partikel ist in Fig. 3 dargestellt. Es umfasst ein kugelförmiges Substrat 100, beispielsweise aus Glas, das mit einer z.B. 5 nm dicken weichmagnetischen Schicht 101 beschichtet ist, die beispielsweise aus einer Eisen-Nickel-Legierung (z. B. Permalloy) besteht. Diese Schicht kann z. B. mit einer Deckschicht 102 überzogen sein, die das Partikel vor Säure schützt. Die zur Sättigung der Magnetisierung solcher Partikel erforderliche Stärke des Magnetfeldes hängt von deren Durchmesser ab. Bei einem Durchmesser von 10 $\mu$m ist dazu ein Magnetfeld von 1 mT erforderlich, während bei einem Durchmesser von 100 $\mu$m ein Magnetfeld von 100 $\mu$T ausreicht. Wenn man eine Beschichtung aus einem Material mit niedrigerer Sättigungsmagnetisierung wählt, erreicht man noch niedrigere Werte.

Fig. 4a und 4b stellen die Magnetisierungskennlinie, d. h den Verlauf der Magnetisierung M als Funktion der Feldstärke H, in einer Dispersion mit solchen Partikeln dar,. Man erkennt, dass sich die Magnetisierung M oberhalb einer Feldstärke +$H_c$ und unterhalb einer Feldstärke -$H_c$ nicht mehr ändert, d. h es liegt eine gesättigte Magnetisierung vor. Zwischen den Werten +$H_c$ und -$H_c$ ist die Magnetisierung nicht gesättigt.

Fig. 4a erläutert die Wirkung eines sinusförmigen Magnetfeldes H(t), wenn kein weiteres Magnetfeld wirksam ist. Die Magnetisierung springt im Takte der Frequenz des Magnetfeldes H(t) zwischen ihren Sättigungswerten hin und her. Der daraus resultierende zeitliche Verlauf der Magnetisierung ist in Fig. 4a mit M(t) bezeichnet. Man erkennt, dass sich die Magnetisierung ebenfalls periodisch ändert, wodurch in einer außerhalb des Spule ein ebenfalls periodisches Signal induziert wird. In Folge der Nichtlinearität der Magnetisierungskennlinie verläuft dieses Signal nicht mehr rein sinusförmig, sondern enthält Oberwellen, d. h höhere Harmonische der sinusförmigen Grundwelle. Diese Oberwellen, die sich leicht von der Grundwelle abtrennen lassen, sind ein Maß für die Partikelkonzentration.

In Fig. 4b ist die Wirkung eines sinusförmigen Magnetfeldes H(t) dargestellt, dem ein statisches Magnetfeld $H_1$ überlagert ist. Da die Magnetisierung dabei in Sättigung ist, wird sie durch das sinusförmige Magnetfeld H(t) praktisch nicht beeinflusst. Die Magnetisierung M(t) bleibt dort zeitlich konstant. Das Magnetfeld H(t) bewirkt also keine Änderung des Magnetisierungszustandes und ruft kein detektiabares Signal hervor, das sich mit einer geeigneten Spulen nachweisen ließe.

Um Aussagen über die räumliche Konzentration der magnetischen Partikel im Untersuchungsobjekt 1 gewinnen, befinden sich oberhalb und unterhalb des Patienten 1 bzw. der Tischplatte mehrere Spulenpaare, deren Wirkungsbereich den Untersuchungsbereich definiert (Fig. 1). Ein erstes Spulenpaar 3 umfasst die beiden koaxial oberhalb und unterhalb des Patienten angeordneten, identisch aufgebauten Wicklungen 3a und 3b, die von gleich großen Strömen, jedoch mit entgegengesetztem Umlaufsinn durchflossen werden. Das dadurch erzeugte Gradienten-Magnetfeld ist in Fig. 2 mit Hilfe der Feldlinien 300 dargestellt. Es hat in Richtung der (senkrechten) Achse des Spulenpaares einen nahezu konstanten Gradienten, und in einem Punkt auf dieser Achse erreicht es den Wert Null. Von diesem feldfreien Punkt ausgehend nimmt die Stärke des Magnetfeld in allen drei Raumrichtungen mit zunehmendem Abstand zu. In einem gestrichelt angedeuteten Bereich 301 (dem ersten Teilbereich) um den feldfreien Punkt herum ist die Feldstärke so gering, dass die Magnetisierung von dort befindlichen magnetischen Partikeln nicht gesättigt ist, während sie außerhalb des Bereichs 301 in einem Zustand der Sättigung ist. In dem außerhalb von 301 verbleibenden Bereich (dem zweiten Teilbereich 302) befindet sich die Magnetisierung der Partikel im Zustand der Sättigung.

Die Größe des die räumliche Auflösung des Gerätes bestimmenden Bereiches 301 hängt einerseits von der Stärke des Gradienten des Gradienten-Magnetfeldes ab und andererseits von der Größe des für eine Sättigung erforderlichen Magnetfeldes. Dieses beträgt 1 mT bei einem Durchmesser der in Fig. 3 dargestellten Kugel von 10 $\mu$m und 100 $\mu$T bei einem Durchmesser von 100 $\mu$m. Bei dem letztgenannten Wert und einem Gradienten des Magnetfeldes von 0,2 T/m hat der Bereich 301, in dem die Magnetisierung der Partikel nicht gesättigt ist, Abmessungen von 1 mm.

Überlagert man dem Gradienten-Magnetfeld im Untersuchungsbereich ein weiteres Magnetfeld, dann verschiebt sich der Bereich 301 in Richtung dieses Magnetfeldes wobei die Größe der Verschiebung mit der Stärke des Magnetfeldes zunimmt. Wenn das überlagerte Magnetfeld zeitlich veränderlich ist, ändert sich die Position Bereichs 301 zeitlich und örtlich entsprechend.

Zur Erzeugung dieser zeitlich veränderlichen Magnetfelder für jede beliebige Richtung im Raum sind drei weitere Spulenpaare vorgesehen. Das Spulenpaar 4 mit den Wicklungen 4a und 4b erzeugt ein Magnetfeld, das in Richtung der Spulenachse des Spulenpaares 3a, 3b verläuft, also vertikal. Die beiden Wicklungen werden zu diesem Zweck mit gleichem

Umlaufsinn von gleich großen Strömen durchflossen. Im Prinzip lässt sich der mit diesem Spulenpaar erzielbare Effekt auch dadurch erreichen, dass den entgegengesetzt gleichen Strömen in dem Spulenpaar 3a, 3b gleichsinnige Ströme überlagert werden, wodurch in dem einen Spulenpaar der Strom abnimmt und in dem anderem Spulenpaar zunimmt. Es kann jedoch von Vorteil sein, wenn das zeitlich konstante Gradienten-Magnetfeld und das zeitlich veränderliche vertikale Magnetfeld von getrennten Spulenpaaren erzeugt werden.

Zur Erzeugung von räumlich horizontal in Längsrichtung des Patienten und in einer dazu senkrechten Richtung verlaufenden Magnetfeldern sind zwei weitere Spulenpaare mit den Wicklungen 5a, 5b und 6a, 6b vorgesehen. Würde man zu diesem Zweck Spulenpaare verwenden, die - ebenso wie die Spulenpaare 3a, 3b und 4a, 4b - vom Helmholtz-Typ wären, dann müssten diese Spulenpaare links und rechts vom Untersuchungsbereich bzw. vor und hinter ihm angeordnet sein. Dadurch würde die Zugänglichkeit des Untersuchungsbereichs erschwert.

Deshalb sind die Wicklungen 5a, 5 b und 6a, 6b der Spulenpaare ebenfalls oberhalb und unterhalb des Untersuchungsbereichs angeordnet, und deshalb müssen sie einen anderen Windungsverlauf haben als das Spulenpaar 4a, 4b. Solche Spulen sind jedoch von Magnetresonanzgeräten mit offenem Magneten (open MRI) bekannt, bei denen sich oberhalb und unterhalb des Untersuchungsbereichs ein HF-Spulenpaar befindet, das ein horizontales zeitlich veränderliches Magnetfeld erzeugen kann. Deshalb kann an dieser Stelle auch auf ein näheres Eingehen auf den Aufbau dieser Spulen verzichtet werden.

Schließlich ist in Fig. 1 noch eine weitere Spule 7 dargestellt, die dazu dient, im Untersuchungsbereich erzeugte Signale zu detektieren. Im Prinzip konnte dazu jedes der felderzeugenden Spulenpaare 3 bis 6 verwendet werden. Jedoch hat die Verwendung einer gesonderten Empfangsspule Vorteile. Es ergibt sich ein günstigeres Signal-Rausch-Verhältnis (insbesondere wenn mehrere Empfangsspulen verwendet werden), und die Spule kann so angeordnet und geschaltet werden, dass sie von den anderen Spulen entkoppelt ist.

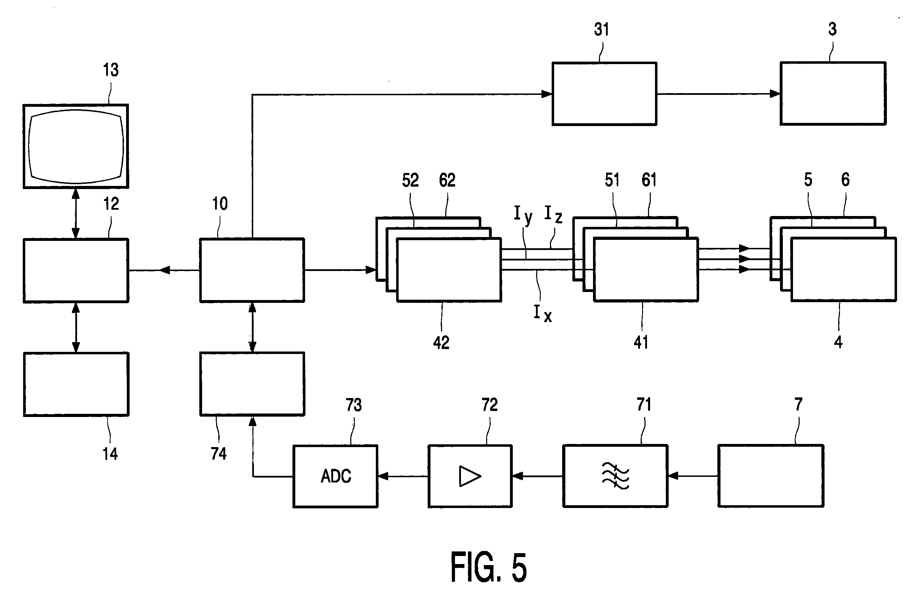

Fig. 5 zeigt ein Prinzipschaltbild des Gerätes nach Fig. 1. Das schematisch dargestellte das Spulenpaar 3 (die Anhänge a, b sind in Fig. 5 bei allen Spulenpaaren der Einfachheit halber weggelassen) wird von einer steuerbaren Stromquelle 31 mit einem Gleichstrom versorgt, der von der Steuereinheit 10 steuerbar - und ein- und ausschaltbar ist. Die Steuereinheit 10 arbeitet mit einer Werkstation 12 zusammen, die mit einem Monitor 13 zur Wiedergabe von die Verteilung der Partikel im Untersuchungsbereich darstellenden Bildern versehen ist. Über eine Tastatur oder ein anderes Eingabegerät 14 sind Eingaben durch einen Benutzer möglich.

Die Spulenpaare 4,5,6 erhalten ihre Ströme von Stromverstärkern 41, 51 und 61. Der zeitliche Verlauf der zu verstärkenden Ströme $I_x$, $I_y$ und $I_z$, die die gewünschten Magnetfelder hervorrufen, wird von je einem Waveform-Generator 42, 52 bzw. 62 vorgegeben. Die Waveform-Generatoren 42, 52, 62 werden von der Steuereinheit 10 gesteuert, die den für das jeweilige Untersuchungsverfahren erforderlichen zeitlichen Verlauf der Ströme berechnet und in die Waveform-Generatoren lädt. Bei der Untersuchung werden diese Signale aus den Waveform-Generatoren ausgelesen und den Verstärkern 41, 51,61 zugeführt, die daraus die für die Spulenpaare 4, 5 und 6 erforderlichen Ströme erzeugen.

Im allgemeinen besteht zwischen der Verschiebung des Bereiches 301 aus seiner Position im Zentrum der Gradientenspulen-Anordnung 3 und dem Strom durch die Gradientenspulen-Anordnung ein nichtlinearer Zusammenhang. Außerdem müssen in der Regal alle drei Spulen ein Magnetfeld erzeugen, wenn der Bereich 301 entlang einer außerhalb des Zentrums verlaufenden Geraden verschoben werden soll. Dies wird bei der Vorgabe des zeitlichen Verlaufs der Ströme durch die Steuereinheit berücksichtigt, beispielsweise mit Hilfe von geeigneten Tabellen. Der Bereich 301 kann daher auf beliebig geformten Wegen durch den Untersuchungsbereich geschoben werden.

Die von der Spule 7 empfangenen Signale werden über ein geeignetes Filter 71 einem Verstärker 72 zugeführt. Die Ausgangssignale des Verstärkers 72 werden von einem Analog-Digital-Wandler 73 digitalisiert und einer Bildverarbeitungseinheit 74 zugeführt, die aus den Signalen und der Position, die der Bereich 301 während des Empfangs der Signale jeweils einnimmt, die räumliche Verteilung der Partikel rekonstruiert.

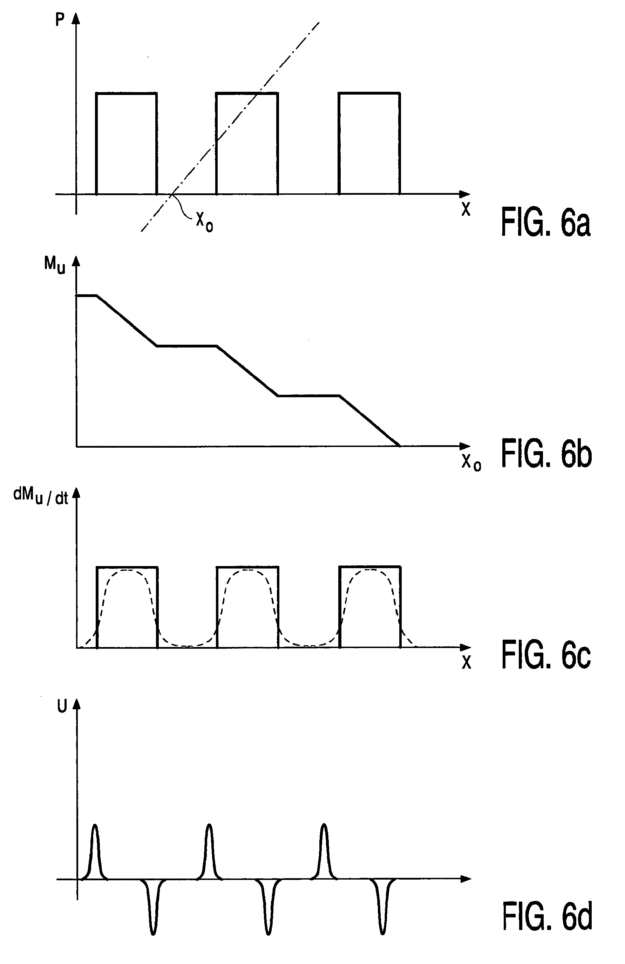

Im folgenden wird anhand der Fig. 6 erläutert, wie die für die Rekonstruktion der Konzentration der Partikel in einem eindimensionalen und sich in x-Richtung erstreckenden Objekt notwendigsn Signale erfasst werden können. In Fig 6a ist die Konzentration P der Partikel in x-Richtung aufgetragen. Der Einfachheit halber ist angenommen, dass es drei gleich breite Bereiche mit derselben Partikelkonzentration gibt und dazwischen Bereiche ohne Partikel. Weiterhin ist angenommen, dass sich das Magnetfeld in x-Richtung linear ändert, wie durch eine gestrichelten Linie angedeutet, wobei es im Punkt $x = x_0$ seine Richtung umkehrt (dort befindet sich also jeweils das Zentrum des Bereiches 301). Schließlich ist angenommen, dass dieser Punkt mit konstanter Geschwindigkeit in x-Richtung verschoben werde.

In Fig. 6b ist die mit diesen Annahmen resultierende Gesamtmagnetisierung $M_u$ im Untersuchungsbereich dargestellt. Dafür gilt die Beziehung

$$M_u = C \int_{-\infty}^{+\infty} f(x - x_o) P(x) dx \qquad (1)$$

Dabei ist C eine Konstante, P(x) die Konzentration der Partikel am Orte x und f(x-$x_0$) eine Funktion, die entsprechend der Magnetisierungskennlinie (vgl. 4a und 4b) den räumlichen Verlauf der Magnetisierung in x-Richtung wiedergibt. Im Idealfall, d. h. wenn die Stärke des Magnetfeldes, die erforderlich ist, um die Magnetisierung zu sättigen, gegen Null tendiert, ist f(x-$x_0$) = -1 für x < $x_0$ und = +1 für x > $x_0$.

Dann ergibt sich der in Fig. 6b dargestellte Verlauf. Dabei ist die Gesamtmagnetisierung $M_u$ außerhalb der Bereiche, in denen Partikel konzentriert sind konstant, und innerhalb dieser Bereiche ändert sie sich entsprechend dem Integral über die Partikelkonzentration. Aus dem räumlichen Verlauf der Gesamtmagnetisierung kann daher die Partikelkonzentration (durch Differentiation) ermittelt werden. Dies setzt voraus, dass man in genügend vielen Positionen $x_0$ die Gesamtmagnetisierung misst, beispielsweise mit Hilfe eines SQUID. Eine solche Messung wäre sehr aufwändig.

Leichter als die Gesamtmagnetisierung lässt sich die zeitliche Ableitung d$M_u$/dt ermitteln, und zwar mit der Empfangsspule 7. Im Idealfall hat das Signal den in Fig. 6c in ausgezogenen Linien dargestellten Verlauf als Funktion des Ortes $x_0$ bzw. (wegen der konstanten Verschiebegeschwindigkeit) als Funktion der Zeit. Wegen der nicht idealen Magnetisierungs-Kennlinie (d h. weil das Magnetfeld erst eine gewisse Stärke haben muss, bevor die Partikel in die Sättigung geraten) ergibt sich jedoch der gestrichelt angedeutete Verlauf. Die scharfen Kanten des Konzentrationsprofils werden dann durch das empfangene Signal nicht mehr zutreffend abgebildet.

Dieser unerwünschte Verlauf ist das Ergebnis der Faltung der Magnetisierung M(x) mit der Funktion f(x-$x_0$) in Gleichung 1. Da die Funktion f(x-$x_0$) durch die magnetischen Eigenschaften der Partikel vorgegeben ist, kann die Faltungsoperation in der Bildverarbeitungseinheit 74 (Fig. 5) durch eine Rückfaltung mit dieser Funktion kompensiert werden. Auch bei einer nicht idealen Magnetisierungs-Kennlinie würde sich dann der in Fig. 6c in ausgezogenen Linien dargestellte Verlauf ergeben.

Das in der Spule 7 induzierte Signal ist umso größere, je schneller die Magnetisierung im Untersuchungsbereich geändert wird Es ist jedoch schwierig, den Bereich 301 schnell durch den gesamten Untersuchungsbereich zu verschieben. Es ist jedoch möglich, dem sich räumlich linear (und langsam) ändernden Magnetfeld, dessen Nullpunkt $X_0$ in x-Richtung verschoben wird, im gesamten Untersuchungsbereich ein zeitlich schnell (z.B. mit einer Frequenz von 200 kHz), vorzugsweise sinusförmig veränderliches Magnetfeld zu überlagern. Dadurch ändert sich die Magnetisierung in dem Bereich in Abhängigkeit von dem überlagerten Magnetfeld, wie in Verbindung mit Fig. 4a und 4b erläutert.

Die Amplitude des dabei in der Spule 7 induzierten Signals als Funktion des Ortes (bzw. der Zeit) hat dann den in Fig. 6d dargestellten Verlauf. Nur wenn sich der Bereich 301 in der Nähe der Kanten des Konzentrationsprofils befindet, ergibt sich eine nennenswerte Signalamplitude. Die Amplitude entspricht daher der räumlichen Ableitung der Partikel-Konzentration. Deshalb ist in diesem Fall in der Bildverarbeitungseinheit 74 noch eine Integration über die Amplitude erforderlich.

Das sinusförmige Magnetfeld, das die Magnetisierungsänderung im Bereich 301 hervorruft, ist gleichzeitig mit dieser Magnetisierungsänderung wirksam, und zwar im gesamten Untersuchungsbereich. Wenn nicht sichergestellt ist, dass die Spule, mit der das sinusförmige Feld erzeugt wird, und die Empfangsspule 7 induktiv völlig voneinander entkoppelt sind, ruft das zeitlich sinusförmige Magnetfeld in der Empfangsspule 7 stets noch eine (unerwünschte) sinusförmige Komponente hervor, die dem aus der Änderung der Magnetisierung im Bereich 301 resultierenden Signal überlagert ist. Ein weiteres Problem ist, dass auch aus dem Bereich 302 in der Spule 7 ein Signal induziert wird, weil die Magnetisierungs-Kennlinie nicht ideal ist und auch noch im Sättigungsbereich eine von Null verschiedene Steigung hat. Das könnte dadurch berücksichtigt werden, dass von dem in der Empfangsspule 7 induzierten Signal ein bestimmter Wert subtrahiert wird.

Dieses Problem lässt sich aber dadurch vermeiden, dass anstelle der in der Spule induzierten Grundwelle mit der Frequenz des sinusförmigen Signals Oberwellen (höhere Harmonische der Grundwelle) zur Auswertung herangezogen werden. Diese höheren Harmonischen können nämlich nur im Bereich 301 aufgrund der nicht linearen Magnetisierungskennlinie der Partikel entstehen. Dementsprechend ist das Filter 71 (Fig. 5) ein Hochpass oder Bandpass, der nur die höheren Harmonischen der Grundschwingung durchlässt.

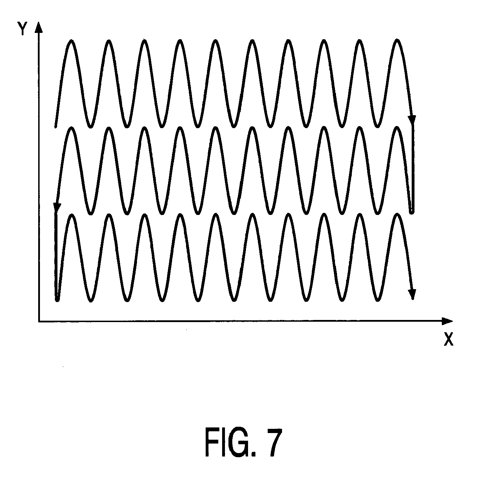

Die Verschiebung des Bereichs 301 in x-Richtung erlaubt lediglich die Ermittlung der räumlichen Verteilung der Partikel in x-Richtung In der Praxis will man diesen Verlauf aber auch in einem zwei- oder dreidimensionalen Bereich ermitteln. Zu diesem Zweck ist dem Magnetfeld, das die Position des Bereichs 301 vergleichsweise langsam in x-Richtung verändert, ein Magnetfeld überlagert, das diese Position in y-Richtung periodisch, z.B sinusförmig verändert - und zwar wesentlich schneller, aber mit kleinerer Amplitude als in x-Richtung. Wenn eine bestimmte Position in x-Richtung erreicht worden ist, wird die Verschiebung in der x-Richtung umgekehrt (der Bereich 301 wird also zurückgsschoben), und gleichzeitig wird das sinusförmige Feld um einen konstanten Wert geändert, so dass sich die in Fig. 7 dargestellte zweidimensionale Verschiebung des Bereichs 301 durch den Untersuchungsbereich ergibt. Überlagert man diesem Feld nach jeder Abtastung des zweidimensionalen Bereichs noch jeweils eine Komponente, die das Magnetfeld in z-Richtung verschiebt, dann kann damit die räumliche Verteilung der Partikel in einem dreidimensionalen Bereich ermittelt werden.

Im Falle der dreidimensionalen Abtastung des Untersuchungsbereiches bzw. eines dreidimensionalen Objektes geht Gleichung (1) über in

$$\mathbf{M}_u = \int_V f(r-r_0) P(r) dV \tag{2}$$

Die fett gedruckten Größen sind Vektoren: $\mathbf{M}_u$ stellt dabei den Vektor der Gesamtmagnetisierung dar, V bezeichnet den Untersuchungsbereich, r und $r_0$ sind die Ortsvektoren eines beliebigen Punktes bzw. des feldstärkefreien Punktes im Untersuchungsbereich. $f(r-r_0)$ ist eine (vektorielle) Funktion, die entsprechend der Magnetisierungskennlinie den räumlichen Verlauf der Magnetisierung wiedergibt, und für die die Beziehung gilt

$$f(r-r_0) = f(|\mathbf{H}(r)|) \cdot E(H(r)) \tag{3}$$

wobei H(r) die magnetische Feldstärke und E(H(r)) den Einheitsvektor in Richtung der magnetischen Feldstärke darstellt. Aus Gleichung (2) lässt sich die Konzentration P(r) Partikel am Ort r durch eine Rückfaltungsoperation in der Bildverarbeitungseinheit 74 (Fig. 5) ermitteln.

Wenn man zwecks Verbesserung der Rekonstruktion nicht nur eine Komponente des Magnetisierungsvektors $\mathbf{M}_u$ erfassen will, sondern in allen drei Raumrichtungen eine Komponente, benötigt man für jede Richtung (mindestens) eine Empfangsspule, die die entsprechende Komponente empfangen kann.

Der Vorteil des erfindungsgemäßen Verfahrens gegenüber Magnetresonanz-Verfahren besteht darin, dass es keinen Magneten erfordert, der ein starkes, räumlich homogenes Magnetfeld erzeugt. Die Anforderungen an die zeitliche Stabilität und die Linearität sind wesentlich geringer als bei dem Magnetresonanz-Verfahren, weshalb der Aufbau eines solchen Geräts wesentlich einfacher sein kann als bei einem MR-Gerät. Die Anforderungen an den räumlichen Verlauf des Magnetfeldes sind ebenfalls geringer, so dass auch Spulen mit "Eisenkernen" (weichmagnetischem Kern, z.B. Eisen) eingesetzt werden können, wodurch sie effektiver und kleiner werden.

Anstelle der in Verbindung mit Fig. 3 erläuterten magnetischen Partikel mit einem weichmagnetischen Überzug können sogenannte Monodomänen-Partikel aus ferro- oder ferrimagnetischem Material verwendet werden. Diese Partikel haben Abmessung im Nanometerbereich und sind so klein, dass sich darin keine magnetischen Domänen bzw Weiß'schen Bereiche ausbilden können. Diese Partikel können in einer geeigneten kolloidalen Dispersion in die Blutbahn eines Patienten injiziert werden. Derartige Dispersionen werden im MR-Bereich bereits als Kontrastmittel injiziert. Die dort verwendeten magnetischen Partikel haben eine Größe von 5 bis 10 nm. Diese Größe ist im Sinne der Erfindung noch nicht optimal. Die zur Sättigung erforderliche magnetische Feldstärke nimmt nämlich mit $1/d^3$ ab, wobei d der Partikeldurchmesser ist. Deshalb sollten die Abmessungen dieser Partikel möglichst groß sein, jedoch nicht so groß, dass sich in ihnen magnetische Domänen ausbilden können. Je nach magnetischem Material liegt die optimale Größe bei einem Wert zwischen 20 und 800 nm.

Die Partikel reichern sich in unterschiedlichen Gewebetypen in unterschiedlichem Maße an. Dieser Effekt kann ebenfalls zur Diagnose ausgenutzt und noch dadurch verstärkt werden, dass die Partikel mit einer Hülle aus organischen Molekülen umgeben werden, die die Bioverträglichkeit erhöhen und bestimmte Adhäsionseigenschaften aufweisen, um sich an bestimmten biologischen Strukturen anzureichern. Die Abbildung der Verteilung dieser Partikel ermöglicht ein sogenanntes "Molecular Imaging" dar.

Magnetische Partikel mit niedriger effektiver Anisotropie haben den Vorteil, dass bei einer Änderung der Magnetisierungsrichtung das einzelne Partikel seine Orientierung nicht ändern muss, weil sich der Magnetisierungsvektor innerhalb des Partikels ändert. Bei Partikeln mit großer effektiver Anisotropie ändert sich die Magnetisierungsrichtung zum Teil innerhalb des Partikels, zum Teil aber auch dadurch, dass sich das Partikel in Richtung des Magnetfeldes ausrichtet. Diese Ausrichtung erfolgt - im Vergleich zur Änderung der Magnetisierungsrichtung innerhalb des Partikels - langsam, wobei die Änderungsgeschwindigkeit von der Viskosität des Mediums abhängt, in dem sich das Partikel befindet.

Dies kann zur Messung der Viskosität (oder der Adhäsion der Partikel) benutzt werden. Dazu wird der Bereich 301 mindestens zweimal mit unterschiedlicher Geschwindigkeit an einen Messpunkt oder Messbereich verschoben, in dem die Viskosität bestimmt werden soll. Die Differenz der für den Messpunkt ermittelten Magnetisierung ist ein Maß für die Viskosität und/oder die Adhäsion Dieser Effekt kann auch zur Messung der Geschwindigkeit der Strömung eines die Partikel enthaltenden Mediums ausgenutzt werden, indem der Bereich 301 mindestens zweimal aus unterschiedlichen Richtungsn an einen Messpunkt oder Messbereich verschoben wird, in dem die Strömungsgeschwindigkeit bestimmt werden soll.

Das erfindungsgemäße Verfahren kann auch in Kombination mit einer MR-Untersuchung durchgeführt werden, wobei die wenigstens einige der vorhandenen Spulen zum Empfang oder zum Empfangen magnetischer Signale benutzt werden können.

**Patentansprüche**

1. Verfahren zur Ermittlung der räumlichen Verteilung magnetischer Partikel in einem Untersuchungsbereich mit den Schritten

   a) Erzeugung eines Magnetfeldes mit einem solchen räumlichen Verlauf der magnetischen Feldstärke, dass sich in dem Untersuchungsbereich ein erster Teilbereich (301) mit niedriger magnetischer Feldstärke ergibt, in welchem die Magnetisierung der magnetischen Partikel nicht gesättigt ist, und ein zweiter Teilbereich (302) mit höherer magnetischer Feldstärke ergibt, in welchem die Magnetisierung der magnetischen Partikel im Zustand der Sättigung ist,
   b) Veränderung der räumlichen Lage der beiden Teilbereiche in dem Untersuchungsbereich, so dass die Magnetisierung der Partikel sich örtlich ändert,
   c) Erfassung von Signalen, die von der durch diese Veränderung beeinflussten Magnetisierung im Untersuchungsbereich abhängen,
   d) Auswertung der Signale zur Gewinnung von Information über die räumliche Verteilung der magnetischen Partikel im Untersuchungsbereich.

2. Verfahren nach Anspruch 1, wobei zur Veränderung der räumlichen Lage der beiden Teilbereiche im Untersuchungsbereich ein örtlich und zeitlich veränderliches Magnetfeld erzeugt wird.

3. Verfahren nach Anspruch 1, wobei die durch die zeitliche Änderung der Magnetisierung im Untersuchungsbereich in wenigstens einer Spule induzierten Signale empfangen und zur Gewinnung von Information über die räumliche Verteilung der magnetischen Partikel im Untersuchungsbereich ausgewertet werden.

4. Verfahren nach Anspruch 3, wobei ein zeitlich veränderliches Magnetfeld in einem ersten Frequenzband auf den Untersuchungsbereich einwirkt und von dem in der Spule empfangenen Signal ein zweites Frequenzband, das höhere Frequenzkomponenten enthält als das erste Frequenzband, zur Gewinnung von Information über die räumliche Verteilung der magnetischen Partikel ausgewertet wird.

5. Verwendung von Monodomänen-Partikeln aus ferro- oder ferritmagnetischen Material in einem Verfahren nach Anspruch 1.

6. Verwendung von Multidomänen-Partikeln aus ferro- oder ferritmagnetischen Material in einem Verfahren nach Anspruch 1.

7. Verwendung von Substraten mit Abmessungen im $\mu$m-Bereich, die mit einer im Vergleich zu diesen Abmessungen dünnen Schicht aus einem Ferromagnetisch weichen Material versehen sind, als Multidomänen-Partikeln nach Anspruch 6.

8. Verwendung der Partikel nach Anspruch 5 oder 6 in einer kolloidalen Dispersion.

9. Anordnung zur Durchführung des Verfahrens nach Anspruch 1 mit

   a) Mitteln zur Erzeugung eines Magnetfeldes mit einem solchen räumlichen Verlauf der magnetischen Feldstärke, dass sich in dem Untersuchungsbereich ein erster Teilbereich (301) mit niedriger magnetischer Feldstärke ergibt, in welchem die Magnetisierung der magnetischen Partikel nicht gesättigt ist, und ein zweiter Teilbereich (302) mit höherer magnetischer Feldstärke ergibt, in welchem die Magnetisierung der magnetischen Partikel im Zustand der Sättigung ist,
   b) Mitteln zur Veränderung der räumlichen Lage der beiden Teilbereiche in dem Untersuchungsbereich, sodass die Magnetisierung der Partikel sich örtlich ändert

c) Mitteln zur Erfassung von Signalen, die von der durch die Veränderung der räumlichen Lage beeinflussten Magnetisierung im Untersuchungsbereich abhängen,

d) Mitteln zur Auswertung der Signale zur Gewinnung von Information über die räumliche Verteilung der magnetischen Partikel im Untersuchungsbereich.

10. Anordnung nach Anspruch 9, wobei die Mittel zur Erzeugung des Magnetfeldes eine Gradientenspulenanordnung zur Erzeugung eines magnetischen Gardientenfeldes umfassen, das in dem ersten Teilbereich des Untersuchungsbereiches seine Richtung umkehrt und einen Nulldurchgang aufweist.

11. Anordnung nach Anspruch 9 mit Mitteln zur Erzeugung eines dem magnetischen Gradientenfeld überlagerten zeitlich veränderlichen Magnetfeldes zwecks Verschiebung der beiden Teilbereiche in dem Untersuchungsbereich.

12. Anordnung nach Anspruch 9 mit einer Spulenanordnung zum Empfangen von durch die zeitliche Änderung der Magnetisierung im Untersuchungsbereich induzierten Signalen.

13. Anordnung nach Anspruch 9 mit Mitteln zur Erzeugung eines ersten und wenigstens eines zweiten, dem magnetischen Gradientenfeld überlagerten Magnetfeldes, wobei das erste Magnetfeld zeitlich langsam und mit großer Amplitude veränderlich ist und das zweite Magnetfeld zeitlich schnell und mit niedriger Amplitude veränderlichen ist.

14. Anordnung nach Anspruch 13, wobei die beiden Magnetfelder im Untersuchungsbereich im wesentlichen zueinander senkrecht verlaufen.

15. Anordnung nach Anspruch 11 mit einem der Spulenanordnung nachgeschalteten Filter, das von dem der Spulenanordnung induzierten Signal die Signalkomponenten in einem ersten Frequenzband unterdrückt und die Signalkomponenten in einem zweiten Frequenzband, das höhere Frequenzkomponenten enthält als das erste Frequenzband, durchlässt.

**Claims**

1. A process for determining the spatial distribution of magnetic particles in an examination region using the following steps:

a) generation of a magnetic field with such a spatial variation of the magnetic field strength, that in the examination region a first subregion (301) has a low magnetic field intensity in which the magnetization of the magnetic particles is unsaturated, and a second subregion (302) has a higher magnetic field strength in which the magnetization of the magnetic particles is in a state of saturation;

b) alteration of the spatial position of the two subregions in the examination region so that the magnetization of the particles varies locally;

c) detection of signals which depend on this alteration of magnetization in the examination region;

d) evaluation of the signals to obtain information regarding the spatial distribution of the magnetic particles in the examination region.

2. A method according to claim 1, wherein for varying the spatial position of the two subregions in the examination region, a spatially and temporally varying magnetic field is generated.

3. A process according to claim 1, wherein said temporal alteration of magnetization in the examination region results in induced signals being received in at least one coil, which signals are evaluated to obtain information regarding the spatial distribution of the magnetic particles in the examination region.

4. A process according to claim 3, wherein a temporally varying magnetic field is applied in a first frequency band to the examination region, and from the signal received in the coil a second frequency band, which contains higher frequency components than the first frequency band, is evaluated to obtain information regarding the spatial distribution of the magnetic particles.

5. Use of mono-domain particles of ferromagnetic or ferrite magnetic material in a process according to claim 1.

6. Use of multi-domain particles of ferromagnetic or ferrite magnetic material in a process according to claim 1.

7. Use of substrates with dimensions in the micron range that are provided with a thin layer compared to these dimensions of a ferro-magnetic soft material such as multi-domain particles according to claim 6.

8. Use of the particles according to claim 5 or 6 in a colloidal dispersion.

9. Arrangement for implementing the process according to claim 1 with:

   a) means for the generation of a magnetic field with such a spatial variation of the magnetic field strength, that in the examination region a first subregion (301) has a low magnetic field intensity in which the magnetization of the magnetic particles is unsaturated, and a second subregion (302) has a higher magnetic field strength in which the magnetization of the magnetic particles is in a state of saturation;
   b) alteration of the spatial position of the two subregions in the examination region so that the magnetization of the particles varies locally;
   c) means for the detection of signals which depend on the alteration in spatial position of magnetization in the examination region;
   d) means for evaluation of the signals to obtain information regarding the spatial distribution of the magnetic particles in the examination region.

10. Arrangement according to claim 9, wherein the means for generating the magnetic field comprises a gradient coil arrangement for generating a gradient magnetic field which reverses its direction in the first subregion of the examination region and has a zero crossing.

11. Arrangement according to claim 9 with means for generating a temporally varying magnetic field on the magnetic gradient field for the purpose of displacement of the two subregions in the examination region.

12. Arrangement according to claim 9 with a coil arrangement for receiving signals induced by the temporally altering magnetization in the examination region.

13. Arrangement according to claim 9 with means for generating a first and at least a second magnetic field superimposed on the magnetic gradient field, said first magnetic field being slowly variable over time with a large amplitude, and said second magnetic field being rapidly variable with time with a small amplitude.

14. Arrangement according to claim 13, wherein the two magnetic fields in the examination region are substantially mutually perpendicular.

15. Arrangement according to claim 11 with a coil arrangement filter installed downstream that suppresses the signal components in a first frequency band induced by the coil arrangement, and allows passage of signals in a second frequency band, that contains higher frequency components than the first frequency band.

**Revendications**

1. Procédé pour déterminer la distribution spatiale de particules magnétiques dans un secteur d'examen comprenant les étapes suivantes :

   a) génération d'un champ magnétique ayant une répartition spatiale de l'intensité de champ magnétique telle qu'il en résulte, dans le secteur d'examen, un premier secteur partiel (301) ayant une intensité de champ magnétique basse, dans lequel la magnétisation des particules magnétiques n'est pas saturée, et un deuxième secteur partiel (302) ayant une intensité de champ magnétique plus élevée dans lequel la magnétisation des particules magnétiques est dans l'état de la saturation,
   b) modification de la position spatiale des deux secteurs partiels dans le secteur d'examen de sorte que la magnétisation des particules se modifie localement,
   c) détection de signaux qui dépendent de la magnétisation influencée par cette modification dans le secteur d'examen,
   d) évaluation des signaux afin d'acquérir des informations sur la distribution spatiale des particules magnétiques dans le secteur d'examen.

2. Procédé selon la revendication 1, dans lequel, en vue de la modification de la position spatiale des deux secteurs partiels dans le secteur d'examen, on génère un champ magnétique variable dans le temps et dans l'espace.

**3.** Procédé selon la revendication 1, dans lequel on reçoit les signaux induits par la modification dans le temps de la magnétisation dans le secteur d'examen dans au moins une bobine et on les évalue en vue de l'acquisition des informations sur la distribution spatiale des particules magnétiques dans le secteur d'examen.

**4.** Procédé selon la revendication 3, dans lequel un champ magnétique variable dans une première bande de fréquences agit sur le secteur d'examen et une deuxième bande de fréquences, qui contient des composantes fréquentielles plus élevées que la première bande de fréquences, est évaluée d'après le signal reçu dans la bobine en vue de l'acquisition des informations sur la distribution spatiale des particules magnétiques.

**5.** Utilisation de particules monodomaines d'un matériau magnétique en fer ou en ferrite dans un procédé selon la revendication 1.

**6.** Utilisation de particules multidomaines d'un matériau magnétique en fer ou en ferrite dans un procédé selon la revendication 1.

**7.** Utilisation de substrats, présentant des dimensions dans le domaine des $\mu$m, qui sont dotés d'une couche mince en comparaison à ces dimensions et faite d'un matériau doux ferromagnétique, en tant que particules multidomaines selon la revendication 6.

**8.** Utilisation des particules selon la revendication 5 ou 6 dans une dispersion colloïdale.

**9.** Agencement destiné à l'exécution du procédé selon la revendication 1 comportant :

a) des moyens de génération d'un champ magnétique ayant une répartition spatiale de l'intensité de champ magnétique telle que, dans le secteur d'examen, il en résulte un premier secteur partiel (301) ayant une intensité de champ magnétique basse, dans lequel la magnétisation des particules magnétiques n'est pas saturée, et un deuxième secteur partiel (302) ayant une intensité de champ magnétique élevée dans lequel la magnétisation des particules magnétiques est dans l'état de la saturation,
b) des moyens de modification de la position spatiale des deux secteurs partiels dans le secteur d'examen de sorte que la magnétisation des particules se modifie localement,
c) des moyens de détection de signaux qui dépendent de la magnétisation influencée par la modification de la position spatiale dans le secteur d'examen,
d) des moyens d'évaluation des signaux en vue de l'acquisition d'informations sur la distribution spatiale des particules magnétiques dans le secteur d'examen.

**10.** Agencement selon la revendication 9, dans lequel les moyens de génération du champ magnétique comportent un agencement de bobines de gradient destiné à la génération d'un champ de gradient magnétique qui, dans le premier secteur partiel du secteur d'examen, inverse son sens et comporte un passage par zéro.

**11.** Agencement selon la revendication 9 doté de moyens de génération d'un champ magnétique variable dans le temps superposé au champ de gradient magnétique à des fins de décalage des deux secteurs partiels dans le secteur d'examen.

**12.** Agencement selon la revendication 9 doté d'un agencement de bobines pour la réception des signaux induits par la modification dans le temps de la magnétisation dans le secteur d'examen.

**13.** Agencement selon la revendication 9 doté de moyens de génération d'un premier champ magnétique et d'au moins un deuxième champ superposé au champ de gradient magnétique, dans lequel le premier champ magnétique est variable plus lentement dans le temps et avec une plus grande amplitude tandis que le deuxième champ magnétique est variable rapidement dans le temps et avec une amplitude plus faible.

**14.** Agencement selon la revendication 13, dans lequel les deux champs magnétiques se coupent sensiblement de manière perpendiculaire dans le secteur d'examen.

**15.** Agencement selon la revendication 11 doté d'un filtre, monté en aval de l'agencement de bobines, qui bloque les composantes du signal induit par l'agencement de bobines dans une première bande de fréquences et laisse passer les composantes du signal induit par l'agencement de bobines dans une deuxième bande de fréquences qui contient des composantes fréquentielles plus élevées que la première bande de fréquences.

# FIG. 1

# FIG. 2

FIG. 3

FIG. 4a

FIG. 4b

FIG. 5

EP 1 304 542 B1

FIG. 6a

FIG. 6b

FIG. 6c

FIG. 6d

FIG. 7

**IN DER BESCHREIBUNG AUFGEFÜHRTE DOKUMENTE**

**In der Beschreibung aufgeführte Patentdokumente**

- DE PS19532676 C
- WO 0140790 A1
- US 4136683 A
- US 5384109 A